# FASCICULE DE BREVET EUROPEEN

(11) **EP 1 438 936 B1**
(45) Date de publication et mention de la délivrance du brevet: **21.10.2009**
(21) Numéro de dépôt: 04356004.4
(22) Date de dépôt: 16.01.2004
(51) Int. Cl.: A61F 2/46

(54) **Ancillaire de pose d'un cotyle prothétique pour une prothèse de hanche**
Hilfsinstrument zum Einsetzen einer prothetischen Gelenkpfanne für eine Hüftprothese
Ancillary for implanting a prosthetic acetabular cup for a hip prosthesis

(30) Priorité: 17.01.2003 FR 0300524
(43) Date de publication de la demande: 21.07.2004
(73) Titulaire: TORNIER, 38330 Saint-Ismier (FR)
(72) Inventeur: Tornier, Alain, 38330 Saint Ismier (FR); Berger, Jean-Pierre, 4052 Beaufays (BE); Reynaud, Patrick, 690004 Lyon (FR); Godeneche, Arnaud, 69380 Dommartin (FR); Panisset, Jean-Claude, 38420 Le Versoud (FR); Deroche, Philippe, 71640 Dracy le Fort (FR); Puget, Jean, 31500 Toulouse (FR); Ferreira, André, 69600 Caluire (FR)
(74) Mandataire: Myon, Gérard Jean-Pierre

(56) Documents cités:
- WO-A-94/21199
- WO-A-95/11641
- WO-A-02/074203
- DE-A- 4 215 888
- DE-A- 10 031 271
- FR-A- 2 797 180
- GB-A- 2 299 758
- US-A- 5 169 399
- US-A- 5 540 697
- US-A- 5 571 111

## Description

La présente invention concerne un ancillaire de pose d'un cotyle prothétique dans une cavité anatomique ou prothétique de la hanche d'un patient, du type comportant un manche de manipulation du cotyle, pourvu, dans sa partie distale, d'une tête de préhension du cotyle et, dans sa partie proximale, d'une surface d'application d'une force d'impaction.

Ce type d'ancillaire, communément appelé « ancillaire préhenseur/impacteur », est utilisé lors d'interventions chirurgicales visant à implanter dans la hanche du patient un cotyle rigide, généralement en métal. Une fois le cotyle implanté dans l'os, une cupule en polymère est logée dans le cotyle, cette cupule étant destinée elle-même à recevoir la tête hémisphérique d'un implant fémoral ou du fémur anatomique. La cupule en polymère est soit solidarisée directement au cotyle par coincement de surfaces tronconiques correspondantes portées par la cupule et le cotyle, soit solidarisée à une coquille métallique qui est reçue de manière articulée, sensiblement à la façon d'une rotule, dans le cotyle fixé à l'os. On parle dans ce dernier cas d'un ensemble cotyloïdien à double mobilité.

Un premier exemple de ce type d'ancillaire consiste en un manche rigide à l'extrémité distale duquel est solidarisée à demeure une tête en matière plastique souple, telle que l'ertafluor (marque déposée). Cette tête, de dimensions extérieures légèrement supérieures aux dimensions intérieures du cotyle à implanter, est introduite et coincée dans ce cotyle, permettant sa préhension puis son positionnement jusqu'à l'emplacement d'impaction de la hanche. Le recours à une telle tête monobloc présente cependant de nombreux inconvénients car la matière souple utilisée ne permet pas une préhension efficace si les dimensions intérieures du cotyle ne sont pas adaptées à celles de la tête, ce qui oblige souvent le chirurgien à saisir par une main le cotyle à implanter pour coincer efficacement la tête. Les conditions de stérilité ne sont donc pas toujours respectées. De plus, la matière souple de la tête est souvent difficilement stérilisable. Par ailleurs, une fois l'impaction du cotyle réalisée, il est généralement difficile de dégager la tête coincée dans le cotyle.

Par le document US-5,169,399, on connaît un autre exemple d'un ancillaire dans lequel un manche préhenseur/impacteur est adapté à la pose d'un ensemble prothétique constitué d'un cotyle métallique et d'un insert polymérique, le cotyle et l'insert étant positionnés et impactés simultanément par le manche. Ce dernier présente à son extrémité distale une tête sous la forme d'une pince dont les deux mâchoires sont écartées l'une de l'autre par un ressort de compression. Lorsque le chirurgien rapproche les deux mâchoires l'une de l'autre en comprimant le ressort, la tête peut être introduite à l'intérieur de l'insert polymérique, puis, par relâchement d'une des mâchoires, l'ensemble prothétique formé de l'insert et du cotyle retenu sur l'insert subit un effort de préhension par la tête. Cet ancillaire est complexe à réaliser et à manipuler, l'effort de préhension étant directement dépendant de la force du ressort de compression interposé entre les mâchoires de la tête. De plus, dans la mesure où la zone de contact entre la tête de l'ancillaire et l'ensemble prothétique est formée par la partie d'extrémité de plus grand diamètre de l'insert polymérique, l'effort d'impaction appliqué par le manche à l'ensemble prothétique ne peut atteindre des valeurs élevées sans risquer d'endommager l'insert et le guidage du cotyle par la tête lors de l'impaction est peu fiable. Dans ces conditions, il n'est pas garanti que le chirurgien n'utilise pas une de ses mains pour maintenir l'ensemble prothétique lors de sa préhension par l'ancillaire. Par ailleurs, cet ancillaire n'est pas applicable à la pose d'un ensemble cotyloïdien à double mobilité évoqué plus haut, la préhension de l'insert n'assurant pas la retenue du cotyle à implanter.

Par le document WO-95/11641, on connaît un autre ancillaire du type précité, qui comporte en outre un embout métallique rapporté de façon amovible à l'extrémité distale du manche de manipulation du cotyle à implanter. Un organe déformable de l'embout est constitué de quatre quadrants, à savoir deux quadrants rigides diamétralement opposés et reliés fixement à une partie proximale de l'embout par des ponts de matière, et deux quadrants mobiles diamétralement opposés, reliés chacun à l'un des quadrants rigides par une charnière élastiquement déformable. Le pivotement des deux quadrants mobiles est commandé par le vissage du manche dans la partie proximale de l'embout. L'utilisation de ces deux quadrants mobiles n'assure cependant un coincement efficace du cotyle que dans deux zones de sa face interne diamétralement opposées, autrement dit qu'avec une force de préhension limitée.

Le but de la présente invention est de proposer un ancillaire qui s'adapte sur des cotyles de dimensions et de nature différentes et qui assure à la fois une préhension efficace du cotyle et une bonne application de la force d'impaction, sans gêner le retrait de l'ancillaire une fois la pose réalisée.

A cet effet, l'invention a pour objet un ancillaire présentant les caractéristiques de la revendication 1.

L'utilisation de la couronne radialement déformable permet à la fois de garantir une préhension efficace, le chirurgien pouvant contrôler la retenue de l'embout par la tête du manche, et de transmettre efficacement au moins une partie de l'effort d'impaction jusqu'au cotyle. En disposant d'une série d'embouts de tailles différentes, le chirurgien est à même d'utiliser le même manche de manipulation pour poser des cotyles de dimensions et/ou de nature différentes.

D'autres caractéristiques de cet ancillaire, prises isolément ou selon toutes les combinaisons techniquement possibles, sont énoncées aux revendications 2 à 10.

On propose également ici une méthode de pose d'un cotyle prothétique dans une cavité anatomique ou prothétique de la hanche d'un patient, dans laquelle :
- on utilise, d'une part, un manche de manipulation du cotyle, pourvu, dans sa partie distale, d'une tête de préhension du cotyle et, à son extrémité proximale, d'une surface d'application d'une force d'impaction, et d'autre part, une série d'embouts de dimensions et/ou de géométrie différentes, comportant chacun une couronne souple radialement déformable par rapport à l'axe longitudinal de l'embout, sur laquelle sont formées à la fois une face de coincement du cotyle et une face opposée d'interaction de l'embout avec la tête du manche,
- on sélectionne, parmi la série d'embouts, un embout dont la face de coincement est sensiblement complémentaire de la paroi interne du cotyle à poser,
- on solidarise l'embout à l'extrémité distale du manche,
- on place l'embout dans le cotyle,
- on met l'embout en prise par la tête du manche de façon à provoquer la déformation radiale de la couronne et, par là, la préhension du cotyle par l'embout,
- on positionne le cotyle dans la cavité de la hanche du patient,
- on applique une force d' impaction sur la surface correspondante du manche,
- on dégage la tête de l'embout, et
- on retire l'embout du cotyle posé.

L'invention sera mieux comprise à la lecture de la description qui va suivre, donnée uniquement à titre d'exemple et faite en se référant aux dessins sur lesquels :
- la figure 1 est une vue en perspective éclatée d'un ancillaire selon l'invention, et d'un cotyle prothétique ;
- la figure 2 est une coupe longitudinale de l'ancillaire de la figure 1 ;
- la figure 3 est une vue du détail III sur la figure 2 ;
- la figure 4 est une coupe longitudinale d'une variante d'un embout de l'ancillaire de la figure 1 ;
- la figure 5 est une coupe longitudinale d'une partie de l'ancillaire pourvu de l'embout de la figure 4 ; et
- la figure 6 est une coupe longitudinale d'une variante d'un ancillaire selon l'invention.

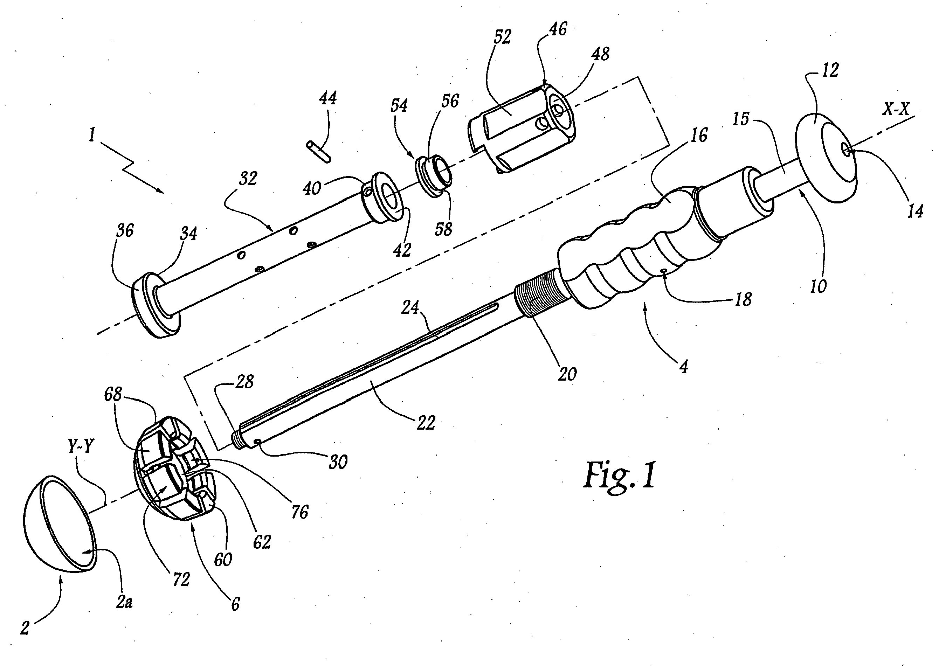

Sur les figure 1 à 3 est représenté un ancillaire 1 de pose d'un cotyle prothétique métallique 2. Cet ancillaire comporte essentiellement un manche 4 s'étendant autour d'un axe X-X et un embout rapporté 6 adapté pour être solidarisé de manière amovible à l'extrémité distale du manche. Le cotyle 2 comporte une paroi interne 2a sensiblement hémisphérique et est destiné à former, avec une coquille intérieure non représentée, un ensemble cotyloïdien à double mobilité évoqué précédemment.

Le manche 4 comporte une tige rigide 10 à l'extrémité proximale de laquelle est fixé rigidement un pommeau 12. Ce pommeau présente une surface proximale 14 légèrement convexe, formant une surface d'application d'une force d'impaction.

En parcourant la tige 10 depuis son extrémité proximale vers son extrémité distale, la tige comprend un premier tronçon cylindrique 15 sur lequel est rapportée fixement une poignée de manipulation 16, solidarisée à la tige par, par exemple, une goupille conique 18. La tige comprend ensuite un tronçon fileté 20 de plus grand diamètre que le tronçon 15, puis un tronçon lisse 22 de plus petit diamètre et sur la surface extérieure duquel est formée une rainure longitudinale 24. L'extrémité distale de la tige est formée d'un ergot saillant 28 co-axial à l'axe X-X et fileté.

Le manche 4 comprend également un manchon tubulaire 32 mobile par rapport à la tige 10 et disposé co-axialement aux tronçons 20 et 22. Ce manchon est pourvu à son extrémité distale d'une tête 34 de forme extérieure tronconique et convergente vers l'extrémité distale du manche. Cette tête délimite de la sorte une surface de rampe tronconique 36, ainsi qu'une surface distale sensiblement plane 38.

L'extrémité proximale du manchon 32 forme un pied sensiblement cylindrique 40 duquel s'étend radialement un rebord d'extrémité saillant 42.

Le pied 40 est muni d'une goupille cylindrique 44 qui s'étend à l'intérieur d'un évidement sensiblement complémentaire formé suivant une direction radiale à l'axe X-X. La longueur de cette goupille est supérieure à l'épaisseur du pied de sorte qu'une partie de la goupille s'étend à l'intérieur du manchon 32 et est engagée dans la rainure longitudinale 24 de la tige 10. Le manchon 32 est ainsi immobilisé en rotation par rapport à la tige, mais libre de se déplacer suivant un mouvement de translation selon la direction X-X.

L'ancillaire 1 comporte des moyens d'entraînement en translation du manchon 32 par rapport à la tige 10, ces moyens se présentant sous la forme d'un corps tubulaire 46 de forme générale cylindrique et essentiellement disposé co-axialement au tronçon fileté 20. Intérieurement, le corps 46 délimite un taraudage 48 complémentaire du tronçon 20 de la tige 10. A son extrémité distale, le corps 46 est pourvu de deux rebord rentrant 50 diamétralement opposés et écartés l'un de l'autre d'une distance supérieure au diamètre extérieur du rebord d'extrémité 42 du manchon 32. Ces rebords 50 forment de la sorte pour le manchon 32 des crochets d'entraînement suivant la direction X-X. Extérieurement, le corps 46 ménage des méplats 52 assurant une bonne prise en main du corps en vue de le solliciter suivant un mouvement de rotation autour de son axe.

Une bague 54 en matière synthétique, par exemple en polyacétal, est axialement interposée entre le manchon 32 et le corps tubulaire 46. Plus précisément, cette bague comporte un corps cylindrique 56 présentant un filetage extérieur complémentaire du filetage du taraudage 48, et une jupe annulaire 58 de diamètre extérieur supérieur au diamètre de l'évidement 48. La jupe 58 forme une couche protectrice pour le manchon 32 lors de la sollicitation de la tige 10, et donc du corps 46 vissé sur cette tige, par application d'une force d'impaction sur le pommeau 12.

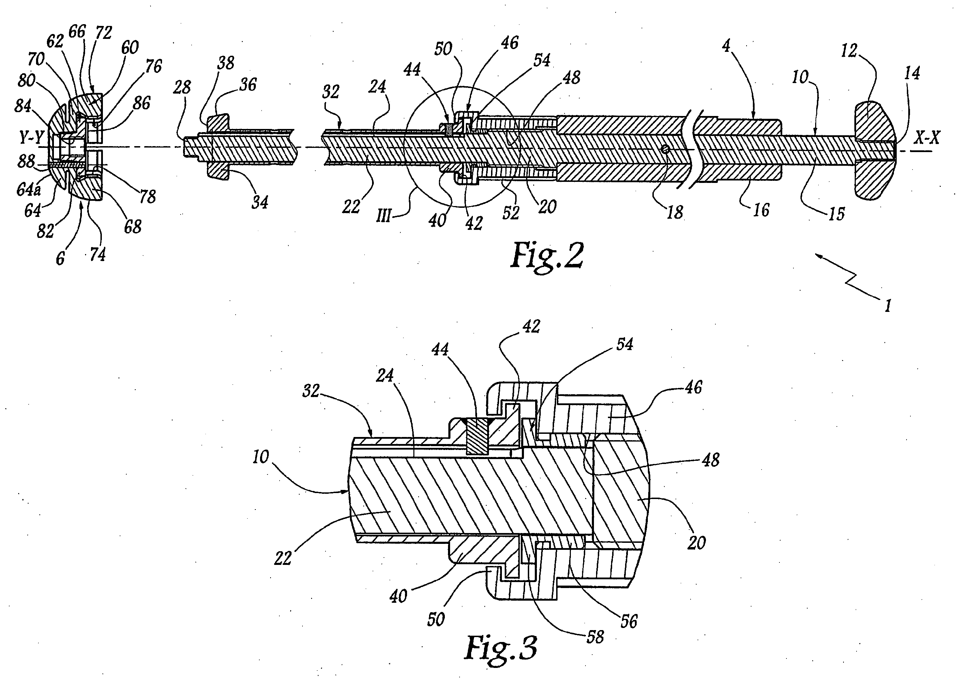

L'embout 6 s'étend quant à lui autour d'un axe Y-Y, co-axial à l'axe X-X sur la figure 2. Il est formé d'un corps 60 souple, constitué par exemple de polyacétal, et d'un insert métallique 62 solidarisé au corps 60 par, par exemple, une goupille 88.

Plus précisément, le corps 60 comporte une base hémisphérique 64 de forme extérieure sensiblement complémentaire à la paroi intérieure 2a du cotyle 2, ainsi qu'une couronne 66 de pétales 68 reliés à la base 64 par des zones 70 de liaison élastiquement déformables. Par déformation simultanée de ces zones 70, la couronne 66 présente une capacité de déformation radiale importante par rapport à l'axe Y-Y. Autrement dit, la couronne 66 est radialement déformable sur sensiblement toute sa périphérie, les pétales 68 étant à même de s'écarter radialement de manière homogène.

A cet effet, la couronne 66 délimite une face 72 de coincement du cotyle 2, constituée des surfaces extérieures 74 sensiblement en forme de tronçon de sphère de chacun des pétales 68, et une face 76 d'interaction de l'embout 6 avec la tête 34 du manchon 32, constituée des surfaces intérieures 78 des pétales 68 qui forment des surfaces de contre-rampe pour la surface de rampe 36 de la tête 34. Chaque surface intérieure 78 comprend une partie cylindrique prolongée vers l'arrière par une partie évasée convergente vers la base 64.

L'insert métallique 62 est formé d'un cylindre 80 d'où s'étend radialement à une de ses extrémités un rebord 82. Le cylindre 80 est vissé dans la base 64 du corps 60 et forme un taraudage 84 complémentaire de l'ergot saillant 28 solidaire de la tige 10. Le rebord 82 forme une surface 86 d'arrêt pour la surface plane 38 de la tête 34.

L'utilisation de l'ancillaire 1 est la suivante :

En considérant le manche 4 à l'état monté représenté sur la figure 2, le chirurgien introduit l'extrémité distale de la tige 10 dans l'embout 6, en rendant co-axial les axes X-X et Y-Y. L'embout 6 est solidarisé au manche 4 par vissage de l'ergot 28 dans le taraudage 84 de l'insert 62. Le chirurgien saisit ensuite l'ancillaire d'une main au niveau de la poignée de manipulation 16 et de son autre main au niveau du corps 46 formant poignée, et entraîne en rotation la poignée 46 par rapport à la tige 10 dans le sens horaire en regardant le manche depuis son extrémité proximale. A la manière d'un système vis-écrou, la poignée 46 pousse le manchon 32 suivant un mouvement de translation selon l'axe X-X, amenant la tête 34 à l'intérieur de la couronne 66 de l'embout. En poursuivant ce mouvement d'entraînement, la surface de rampe 36 coopère avec les surfaces de contre-rampe 78 portées intérieurement par les pétales 68 de façon à dilater radialement la couronne 66 et ainsi plaquer les surfaces extérieures 74 des pétales contre la paroi interne du cotyle 2. En d'autres termes, la couronne 66 passe d'une configuration de repos à une configuration dilatée vers l'extérieur, dont le contour extérieur globalement circulaire est de diamètre supérieur à celui du contour de la couronne dans sa configuration de repos. On comprend que toute la face extérieure 74 de la couronne 66 participe alors au coincement du cotyle 2. On notera à ce propos que, pour l'ancillaire 1 représenté, les pétales 68 de cette couronne sont identiques, ce qui n'est pas une limitation à l'invention dans le sens où il convient surtout de prévoir que la déformation radiale de la couronne par la tête de l'ancillaire entraîne l'appui quasi-simultané de l'essentiel de la surface extérieure de la couronne sur la face interne concave du cotyle à manipuler.

Le chirurgien poursuit ce mouvement jusqu'à ce qu'il estime que la retenue de l'embout par la tête est suffisamment importante pour garantir une bonne préhension par coincement du cotyle par l'embout. La surface d'arrêt 86 portée par l'embout évite tout risque de déformation excessive des pétales, l'insert 62 imposant ainsi une position axiale maximale pour la tête 34.

Le chirurgien manipule alors le cotyle librement à l'aide de l'ancillaire 1, le positionne sur la hanche du patient au niveau d'une cavité anatomique ou bien prothétique, c'est-à-dire formée par une plaque de reconstruction osseuse préalablement implantée. Puis, à l'aide d'un maillet approprié, il applique une force d'impaction sur la surface 14. L'effort d'impaction est transmis suivant la direction X-X de la tige 10 à l'insert rigide 62 qui est vissé sur cette tige, puis de l'insert à la base 64 du corps souple 62, et enfin de cette base au cotyle 2 au travers de la surface extérieure 64a de la base, provoquant l'emmanchement en force du cotyle dans la cavité correspondante de la hanche.

Une fois l'impaction réalisée, le chirurgien entraîne dans un mouvement de rotation inverse la poignée 46 par rapport à la tige 10 de façon à dégager la tête 34 de l'embout 6. Par déformation élastique de rappel des zones de liaison 70, les pétales 68 reprennent leur position initiale, permettant de retirer sans effort l'embout du cotyle implanté.

L'ancillaire selon l'invention permet ainsi de manipuler aisément et avec précision un cotyle prothétique à implanter. Le chirurgien n'est à aucun moment obligé de toucher le cotyle avec ses mains. L'effort d'impaction est efficacement transmis au cotyle, suivant la direction longitudinale du manche 4, et ce à travers la partie souple de l'embout. Les risques de marquer, voire d'endommager le cotyle impacté sont ainsi limités, à la différence d'ancillaires impacteurs relevant de l'art antérieur, tels que ceux envisagés dans US-5,169,399 et WO-95/11641 évoqués plus haut, pour lesquels des parties dures des ancillaires sont directement appuyées sur la tranche du cotyle, autrement dit au niveau d'une zone fragile et d'aire restreinte.

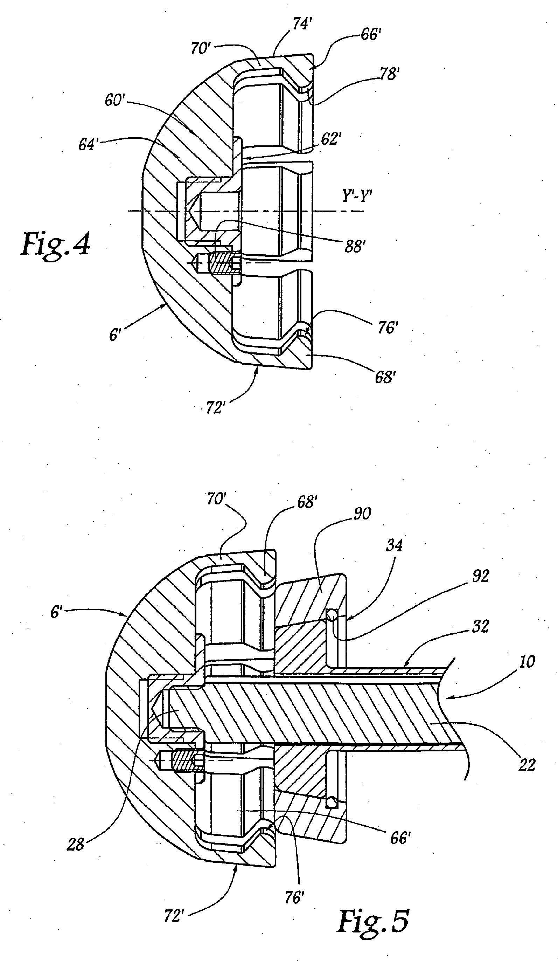

Avantageusement, l'ancillaire selon l'invention comprend une série d'embouts dont les dimensions et les géométries sont différentes. Par exemple, cette série comporte, en plus de l'embout 6, un embout 6', représenté sur la figure 4, de même structure générale que l'embout 6, mais dont la face extérieure du corps souple est conformée pour un cotyle dont la paroi interne est en partie tronconique. Cet embout est adapté pour la préhension et l'impaction de cotyles dans lesquels sont directement solidarisés par coincement un insert polymérique.

Plus précisément, l'embout 6' est essentiellement constitué des mêmes éléments que l'embout 6, ces derniers étant référencés par les mêmes chiffres marqués d'un prime. Ainsi, l'embout 6' comporte un corps 60' en matière souple et un insert rigide 62', rigidement fixés l'un à l'autre par une vis 88'. Outre la géométrie de sa face extérieure, l'embout 6' d'axe Y'-Y' se distingue essentiellement de l'embout 6 par la forme de ses pétales 68' et des zones 70' de liaison avec la base 64' : la face extérieure 74' de chaque pétale 68' est tronconique convergente vers la base, la face intérieure 78' de chaque pétale est évasée sur toute sa longueur et les zones de liaison 70' s'étendent parallèlement à l'axe Y'-Y'.

Comme représenté sur la figure 5, la tête 34 du manche 4 est alors équipée d'une douille 90 solidarisée au reste du manchon 32 par un circlip de retenue 92. Cette douille augmente la dimension extérieure de la tête 34 de façon à adapter cette dernière à l'embout 6' et délimite ainsi latéralement une surface de rampe adaptée pour coopérer avec la face d'interaction 76' de l'embout.

L'utilisation de l'ancillaire 1 muni de l'embout 6' est sensiblement analogue à l'utilisation décrite précédemment. Lors de l'application de l'effort d'impaction, la force est essentiellement transmise au cotyle par les surfaces tronconiques 74' des pétales 68'.

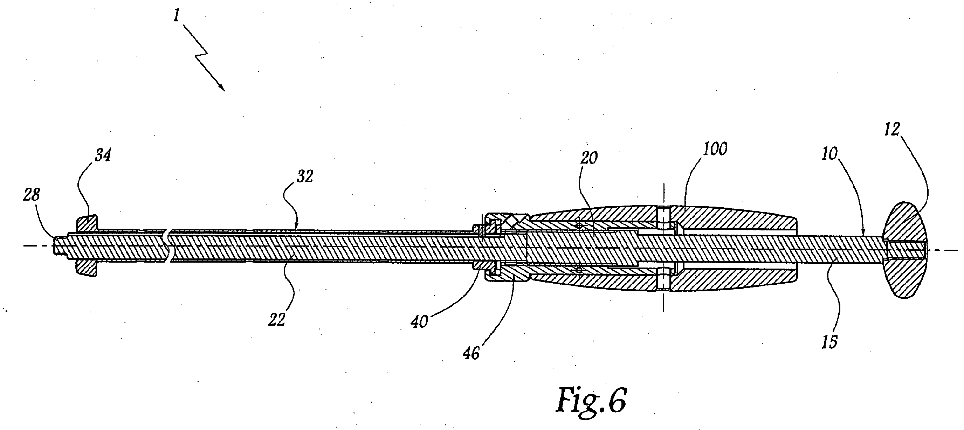

Sur la figure 6 est représentée une variante de l'ancillaire 1 qui se distingue essentiellement de l'ancillaire des figures 1 à 3 par ce qui suit. Contrairement à la poignée de manipulation 16 des figures précédentes qui est solidarisée rigidement à la tige 10, l'ancillaire de la figure 6 comporte une poignée de manipulation 100 qui est solidarisée au corps 46 vissé sur la tige 10. L'utilisation de cette variante est sensiblement analogue à celle de l'ancillaire des figures 1 à 3, l'utilisateur devant, pour provoquer le déplacement en translation de la tête 34 par rapport à la tige 10, appliquer un mouvement de rotation sur l'ensemble corps 46/poignée 100, par exemple en saisissant d'une main la poignée 100 et en saisissant de son autre main le pommeau 12.

Divers aménagements et variantes à l'ancillaire selon l'invention sont également envisageables :
- d'autres matériaux que le polyacétal peuvent être utilisés pour réaliser le corps souple de l'embout 6, les matériaux retenus devant à la fois combiner une bonne résistance au choc en raison de la transmission des efforts d'impaction, ainsi qu'une bonne résistance à la stérilisation, et ne pas endommager la paroi interne du cotyle à implanter ; et/ou
- l'embout rapporté à l'extrémité distale du manche de l'ancillaire est formé d'une pièce d'un seul tenant, par exemple en matière synthétique, formée d'une partie souple comprenant une couronne radialement déformable de manière homogène, analogue à la couronne 66, et d'une partie plus rigide, par exemple en matériau armé ou renforcé, dans laquelle le manche est solidarisable de manière amovible.

## Revendications

1. Ancillaire de pose d'un cotyle prothétique (2) dans une cavité anatomique ou prothétique de la hanche d'un patient, comportant un manche (4) de manipulation du cotyle (2), pourvu, dans sa partie distale, d'une tête (34) de préhension du cotyle et, dans sa partie proximale, d'une surface (14) d'application d'une force d'impaction, et au moins un embout rapporté (6 ; 6'), adapté pour être solidarisé de manière amovible à l'extrémité distale (28) du manche (4) et définissant à la fois une face (72 ; 72') de coincement du cotyle (2) et une face opposée (76 ; 76') d'interaction de l'embout avec la tête (34) du manche, **caractérisé en ce que** l'embout (6 ; 6') comporte une base (64 ; 64') et une couronne (66 ; 66'), laquelle couronne est reliée élastiquement à la base dé manière à être radialement déformable par rapport à l'axe longitudinal (Y-Y) de l'embout et comporte plusieurs pétales (68 ; 68'), les faces de coincement (72 ; 72') et d'interaction (76 ; 76') étant respectivement constituées des surfaces extérieures (74 ; 74') et intérieures (78 ; 78') de chacun ces pétales.

2. Ancillaire suivant la revendication 1, **caractérisé en ce que** la couronne (66 ; 66') est reliée élastiquement à la base (64 ; 64') de manière à être radialement déformable sur sensiblement toute la périphérie de la couronne.

3. Ancillaire suivant l'une quelconque des revendications précédentes, **caractérisé en ce que** l'embout (6 ; 6') comporte à la fois une première partie (60 ; 60') incluant la base (64 ; 64') et la couronne (66 ; 66'), et une seconde partie (62 ; 62'), plus rigide que la première partie, solidaire de la première partie et pourvue de moyens (84) de solidarisation amovible à l'extrémité distale (28) du manche (4).

4. Ancillaire suivant la revendication 3, **caractérisé en ce que** la seconde partie de l'embout (6 ; 6') est constituée d'un insert métallique (62 ; 62') fixé à la première partie (60 ; 60').

5. Ancillaire suivant l'une des revendications 3 ou 4, **caractérisé en ce que** la seconde partie (62 ; 62') de l'embout (6 ; 6') délimite une surface d'arrêt (86), suivant la direction longitudinale (X-X) du manche (4), pour la tête du manche (34).

6. Ancillaire suivant l'une quelconque des revendications 3 à 5, **caractérisé en ce que** la première partie (60 ; 60') de l'embout (6 ; 6') présente au moins une surface (64a ; 74') de transmission de la force d'impaction entre le manche (4) et le cotyle (2).

7. Ancillaire suivant l'une quelconque des revendications précédentes, **caractérisé en ce que** la tête (34) du manche (4) définit une surface de rampe (36) adaptée pour coopérer avec la face (76 ; 76') d'interaction de l'embout (6 ; 6').

8. Ancillaire suivant l'une quelconque des revendications précédentes, **caractérisé en ce que** le manche (4) comporte une tige rigide (10) sur laquelle la tête (34) est montée de façon mobile, ainsi que des moyens (46) d'entraînement de la tête par rapport à la tige suivant un mouvement de translation selon la direction longitudinale (X-X) de la tige.

9. Ancillaire suivant la revendication 8, **caractérisé en ce que** le manche (4) comporte un manchon (32) disposé co-axialement à la tige (10) et à l'extrémité distale duquel est rigidement fixée la tête (34), et **en ce que** les moyens d'entraînement de la tête par rapport à la tige comportent une poignée (46) vissée sur la tige et liée en translation avec le manchon (32), ce manchon étant immobilisé en rotation par rapport à la tige.

10. Ancillaire suivant la revendication 9, **caractérisé en ce qu'**une bague (54) est axialement interposée entre le manchon (32) et la poignée vissée (46).

## Claims

1. Ancillary for implanting a prosthetic acetabular cup (2) into an anatomical cavity or hip prosthesis of a patient, comprising a handle (4) for manipulating the acetabular cup (2), which in its distal part is provided with a head (34) for gripping the acetabular cup and in its proximal part is provided with a surface (14) for applying an impaction force, and at least one cap is applied (6; 6'), which is adapted to be interlocked in a removable way with the distal end (28) of the handle (4), defining at the same time a face (72; 72') for wedging the acetabular cup (2) and an opposite face (76; 76') for the cap to interact with the head (34) of the handle, **characterised in that** the cap (6; 6') has a base (64; 64') and a crown (66; 66'), which crown is connected elastically to the base so that it may be distorted radially in relation to the longitudinal axis (Y-Y) of the cap and comprises several lobes (68; 68'), the faces for wedging (72; 72') and interacting (76; 76') being made up respectively of the external surfaces (74; 74') and internal surfaces (78; 78') of each of these lobes.

2. Ancillary according to claim 1, **characterised in that** the crown (66; 66') is connected elastically to the base (64; 64') so that it may be distorted radially over more or less the whole periphery of the crown.

3. Ancillary according to any one of the previous claims, **characterised in that** the cap (6; 6') comprises at the same time a first part (60; 60'), including the base (64; 64') and the crown (66; 66'), and a second part (62; 62'), which is more rigid than the first part, interlocking with the first part and provided with means (84) of interlocking in a removable way at the distal end (28) of the handle (4).

4. Ancillary according to claim 3, **characterised in that** the second part of the cap (6; 6') is made up of a metal insert (62; 62'), which is fixed to the first part (60; 60').

5. Ancillary according to one of claims 3 or 4, **characterised in that** the second part (62; 62') of the cap (6; 6') delimits a stopping surface (86) for the head of the handle (34) in the longitudinal direction (X-X) of the handle (4).

6. Ancillary according to any one of claims 3 to 5, **characterised in that** the first part (60; 60') of the cap (6; 6') has at least one surface (64a; 74') for transmitting the impaction force between the handle (4) and the acetabular cup (2).

7. Ancillary according to any one of the previous claims, **characterised in that** the head (34) of the handle (4) defines an inclined surface (36), which is adapted to cooperate with the face (76; 76') for interacting with the cap (6; 6').

8. Ancillary according to any one of the previous claims, **characterised in that** the handle (4) comprises a rigid rod (10), on which the head (34) is fitted in a moveable way, as well as means (46) of entrainment of the head in relation to the rod in a translatory movement in the longitudinal direction (X-X) of the rod.

9. Ancillary according to claim 8, **characterised in that** the handle (4) comprises a sleeve (32), which is arranged coaxially to the rod (10), at the distal end of which the head (34) is fixed rigidly, and **in that** the means of entrainment of the head in relation to the rod comprise a handgrip (46), which is screwed onto the rod and connected in translation with the sleeve (32), this sleeve being immobilised in rotation in relation to the rod.

10. Ancillary according to claim 9, **characterised in that** a ring (54) is interposed axially between the sleeve (32) and the handgrip (46), which is screwed on.

## Patentansprüche

1. Hilfsmittel zum Einsetzen einer Gelenkpfannenprothese (2) in eine anatomische oder prothetische Aushöhlung der Hüfte eines Patienten, umfassend einen Stiel (4) zum Handhaben der Gelenkpfanne (2), der in seinem distalen Teil mit einem Greifkopf (34) für die Gelenkpfanne und in seinem proximalen Teil mit einer Anlegefläche (14) für das Aufbringen einer Einsetzkraft versehen ist, sowie mindestens einen angebauten Ansatz (6; 6'), der mit dem distalen Ende (28) des Stiels (4) lösbar verbindbar ist und der gleichzeitig eine Klemmseite (72; 72') für die Gelenkpfanne (2) und eine entgegen gesetzte Seite (76; 76') zur Wechselwirkung des Ansatzes mit dem Kopf (34) des Stiels definiert, **dadurch gekennzeichnet, dass** der Ansatz (6; 6') eine Basis (64; 64') und einen Kranz (66; 66') aufweist, wobei der Kranz elastisch mit der Basis derart verbunden ist, dass er radial in Bezug zu der Längsachse (Y-Y) des Ansatzes verformbar ist und mehrere Blätter (68; 68') aufweist, wobei die Klemm- (72; 72') und Wechselwirkungsflächen (76; 76') jeweils aus externen Flächen (74; 74') und internen Flächen (78; 78') jedes dieser Blätter bestehen.

2. Hilfsmittel nach Anspruch 1, **dadurch gekennzeichnet, dass** der Kranz (66; 66') elastisch mit der Basis (64; 64') derart verbunden ist, dass er auf im Wesentlichen dem ganzen Umfang des Kranzes radial verformbar ist.

3. Hilfsmittel nach einem der vorhergehenden Ansprüche, **dadurch gekennzeichnet, dass** der Ansatz (6; 6') sowohl einen ersten Teil (60; 60'), der die Basis (64; 64') und den Kranz (66; 66') enthält, als auch einen zweiten Teil (62; 62') aufweist, der starrer ist als der erste Teil, der fest mit dem ersten Teil verbunden und mit Mitteln (84) zum abnehmbaren Verbinden an dem distalen Ende (28) des Stiels (4) versehen ist.

4. Hilfsmittel nach Anspruch 3, **dadurch gekennzeichnet, dass** der zweite Teil des Ansatzes (6; 6') aus einem Metalleinsatz (62; 62') besteht, der an dem ersten Teil (60; 60') befestigt ist.

5. Hilfsmittel nach einem der Ansprüche 3 oder 4, **dadurch gekennzeichnet, dass** der zweite Teil (62; 62') des Ansatzes (6; 6') eine Stoppfläche (86) entlang der Längsrichtung (X-X) des Stiels (4) für den Kopf des Stiels (34) abgrenzt.

6. Hilfsmittel nach einem der Ansprüche 3 bis 5, **dadurch gekennzeichnet, dass** der erste Teil (60; 60') des Ansatzes (6; 6') mindestens eine Fläche (64a; 74') zum Übertragen der Aufprallkraft zwischen dem Stiel (4) und der Gelenkpfanne (2) aufweist.

7. Hilfsmittel nach einem der vorhergehenden Ansprüche, **dadurch gekennzeichnet, dass** der Kopf (34) des Stiels (4) eine Rampenfläche (36) definiert, die mit der Wechselwirkungsseite (76; 76') des Ansatzes (6; 6') zusammenarbeiten kann.

8. Hilfsmittel nach einem der vorhergehenden Ansprüche, **dadurch gekennzeichnet, dass** der Stiel (4) einen starren Schaft (10), auf dem der Kopf (34) beweglich montiert ist, sowie Mittel (46) zum Antreiben des Kopfs in Bezug zu dem Schaft mit einer Verschiebungsbewegung entlang der Längsrichtung (X-X) des Schafts aufweist.

9. Hilfsmittel nach Anspruch 8, **dadurch gekennzeichnet, dass** der Stiel (4) einen Stutzen (32) aufweist, der koaxial zu dem Schaft (10) angeordnet ist, und an dessen distalem Ende der Kopf (34) starr befestigt ist, und dass die Mittel zum Antreiben des Kopfs in Bezug zu dem Schaft einen Griff (46) aufweisen, der auf den Schaft geschraubt und in Verschiebung mit dem Stutzen (32) verbunden ist, wobei dieser Stutzen in Drehung von dem Schaft festgestellt wird.

10. Hilfsmittel nach Anspruch 9, **dadurch gekennzeichnet, dass** ein Ring (54) axial zwischen dem Stutzen (32) und dem geschraubten Griff (46) eingefügt ist.
